# EUROPEAN PATENT SPECIFICATION

(11) **EP 1 962 696 B1**
(45) Date of publication and mention of the grant of the patent: **28.03.2012**
(21) Application number: 06848007.8
(22) Date of filing: 19.12.2006
(51) Int. Cl.: A61B 17/22

(54) **CUTTING BALLOON CATHETER ASSEMBLY**
SCHNEIDEBALLONKATHETER-ANORDNUNG
ENSEMBLE CATHETER A BALLONNET COUPANT

(30) Priority: 20.12.2005 US 751865 P
(43) Date of publication of application: 03.09.2008
(73) Proprietor: Medical Components, Inc., Harleysville, PA 19438 (US); TwinCath, LLC, Paradise Valley, AZ 85253 (US); Mishler, Richard, Phoenix, AZ 85028 (US)
(72) Inventor: SCHON, Donald, M., Paradise Valley, AZ 85253 (US); MISHLER, Richard, Phoenix, AZ 85028 (US); SCHWEIKERT, Timothy, Levittown, PA 19054 (US)
(74) Representative: Adamson Jones
(86) International application number: PCT/US2006/048971
(87) International publication number: WO 2007/075986

(56) References cited:
- EP-A- 1 169 970
- US-A- 3 996 938
- US-A- 5 904 698
- US-A- 6 156 254
- US-A1- 2004 143 287
- US-B1- 6 626 861
- US-B1- 6 635 068

## Description

### FIELD OF THE INVENTION

The present invention relates to medical devices and more particularly to a cutting balloon device for expanding passageways in the venous system.

### BACKGROUND OF THE INVENTION

One of the most common heart diseases in industrialized countries is atherosclerotic cardiovascular disease, caused by the buildup of plaque or stenoses in the blood vessels. This affliction affects not only veins or arteries, but also dialysis access systems such as fistulas or grafts. Generally, arteries are susceptible to the buildup of plaque. The venous system, however, has lesions that are generally fibrous in nature, usually in the form of scar tissue or venous valvular rings. Additionally, when there is long term placement of a fistula or graft, there is a tendency for the passageway to narrow. The venous anastomosis of a graft, which may be made from PTFE or some other suitable material, may develop a different lesion, such as fibro-muscular hyperplasia. All three of these inflictions are distinct in their nature; however, they all generally respond well to balloon dilation or angioplasty.

There are numerous inventions that have attempted to successfully expand venous passageways that have been narrowed by plaque or stenoses. One of the most well known is the "Fogarty catheter," which is described in detail in U.S. Pat. No. 3,435,826 (the `826 patent) to Fogarty, as well as U.S. Pat. No. 4,403,612 (the `612 patent). Both the '826 patent and the `612 patent describe inflatable balloon catheters. The balloon catheter of the `826 patent comprises a catheter having an inflatable balloon at its distal tip. The balloon catheter of the '826 patent is operated by inserting the deflated balloon catheter into the vessel beyond the clogged portion, inflating the balloon and then pulling the inflated balloon towards the clogged area, thereby dislodging the clog and dragging the blockage to an incision where the clog can be removed. The balloon catheter of the `612 patent has two balloons, wherein a first balloon is disposed inside of a second balloon and both the first and second balloon are located at the distal tip of a catheter. In use, the balloon catheter of the `612 patent is inflated and compresses the plaque or stenoses that is located along the wall of a vessel, thereby enlarging the venous passageway.

Most types of inflatable balloon catheters expand the vessel by exerting pressure on the buildup located on the walls of the vessel and squeezing the buildup against the vessel wall. There are other types of balloon catheters that do not inflate; rather, they expand using some other mechanical process. An example of this type of mechanically expanding balloon catheter is described in detail in U.S. Pat, No. 4,921,484 to Hillstead (the '484 patent). The '484 patent describes a catheter having a woven mesh balloon at its distal tip. During insertion, the balloon is elongated and maintains a narrow profile. When the balloon reaches the vessel to be expanded, the distal tip of the catheter is contracted, thereby expanding the woven mesh balloon. The expanded mesh balloon is then used to break through or scrape plaque and stenoses, thereby expanding the vessel.

US 6,626,861 discloses a coaxial dual lunen ballon catheter assembly wherein the ballon comprises an expandable cover having an abrasive textures.

It would be beneficial to provide a device for removing plaque and stenoses from dialysis accesses such as fistulas or grafts, as well as blood vessels that may be clogged, that is inflatable through the injection of a fluid into the center of the balloon and also has the surface characteristics of a mesh balloon.

### SUMMARY OF THE INVENTION

The present invention is a cutting balloon catheter assembly including a dual lumen catheter having an inflatable balloon at its distal end having an interior cavity and an expandable covering disposed about the balloon, wherein the covering has an array of cutting edges. In the assembly, the balloon is fixedly connected to the distal end of the catheter and the interior cavity of the balloon is in fluid communication with an inflation/deflation lumen of the catheter.

### BRIEF DESCRIPTION OF THE DRAWINGS

The accompanying drawings, which are incorporated herein and constitute part of this specification, illustrate the presently preferred embodiments of the invention, and, together with the general description given above and the detailed description given below, serve to explain the features of the invention. In the drawings:

Fig. 1 is a side view of a cutting balloon catheter assembly according to an embodiment of the present invention;

Fig. 2 is an enlarged sectional view of the cutting balloon catheter assembly of Fig. 1, taken along line 2-2, with the balloon inflated;

Fig. 3 is an enlarged side view of a distal end of the cutting balloon catheter assembly of Fig. 1, in a deflated condition;

Fig. 3a is an enlarged sectional view of an alternative embodiment of a cutting balloon catheter assembly;

Fig. 4 is a sectional view of a hub of the catheter cutting balloon assembly of Fig. 1;

Fig. 5 is a side view, partially in section, of the distal end portion of the cutting balloon catheter assembly of Fig. 1, inserted into a patient's vessel and in an inflated condition; and

Fig. 6 is a sectional view of the distal end portion of an alternative embodiment of the cutting balloon catheter assembly.

### DETAILED DESCRIPTION OF THE INVENTION

In the drawings like numerals indicate like elements throughout. Certain terminology is used herein for convenience only and is not to be taken as a limitation on the present invention. The words "proximal" and "distal" refer to directions away from and closer to, respectively, the tip of the double lumen catheter assembly that makes up a portion of the cutting balloon assembly according to the present invention. The terminology includes the words above specifically mentioned, derivatives thereof, and words of similar import. The following describes preferred embodiments of the invention. However, it should be understood based on this disclosure, that the invention is not limited by the preferred embodiments described herein.

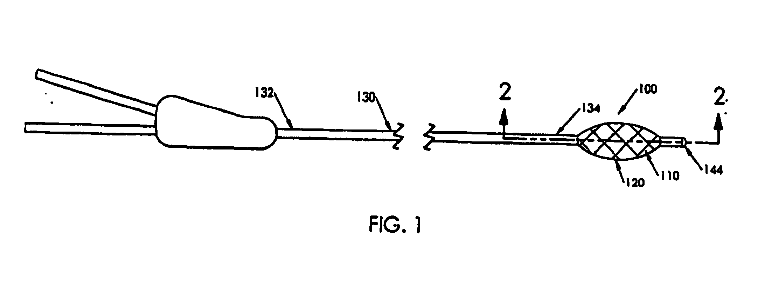

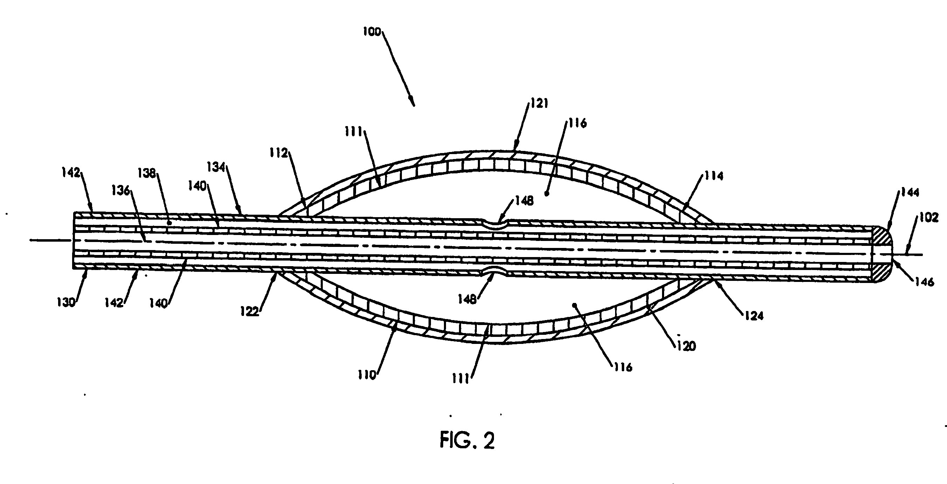

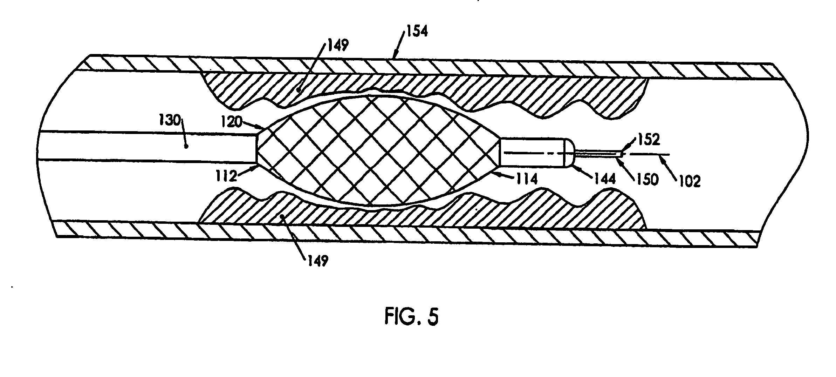

Referring to Figs. 1 and 2, a first preferred embodiment of a cutting balloon catheter assembly 100 according to the present invention is shown. Balloon assembly 100 is used to expand a patient's blood vessel by cutting blockages within the vessel. A blockage may be a lesion, stenoses, plaque or any other infliction that would constrict a patient's vessel. The balloon assembly 100 includes a balloon 110, a mesh covering 120 and a catheter 130. The catheter 130 comprises a proximal end 132, a distal end 134 and a longitudinal axis 102 extending through the catheter 130 between the proximal end 132 and the distal end 134. Preferably, the catheter 130 is a dual lumen coaxial catheter and has an interior wall 140 and an exterior wall 142. In the preferred embodiment shown in Fig. 2, the catheter 130 has an axial lumen 136 located along the longitudinal axis 102, and an exterior or inflation/deflation lumen 13 8 located about the axial lumen 136. The axial lumen 136 is sized to accommodate a guidewire (see Fig. 5) inserted therethrough along the longitudinal axis 102. The axial lumen 136 is preferably defined entirely by the interior wall 140. The exterior lumen 138 is defined by the exterior wall 142 and the interior wall 140. While it is preferable, as shown in Fig. 2, that the axial lumen 136 be centered within the catheter 130 and entirely surrounded by the exterior lumen 138 (i.e., coaxial), those skilled in the art will recognize that the axial lumen 136 may be disposed anywhere within the exterior wall 142 of the catheter 130.

A distal tip 144 is located at the distal end 134 of the catheter 130, at a point that is distal of the balloon 120. Preferably, the distal tip 144 is rounded to facilitate smooth insertion of the assembly 100 into a patient's blood vessel. The distal tip 144 preferably is radiopaque for precision location at a site using known imaging techniques and has a distal passageway 146 that is in fluid communication with the axial lumen 136, and provides a passageway between the axial lumen 136 and the outside of the distal tip 144. The distal passageway 146 facilitates the insertion of the guidewire through the distal end 144 and the axial lumen 136. Preferably, the catheter 130 is constructed of polyurethane or some other suitable biocompatible material.

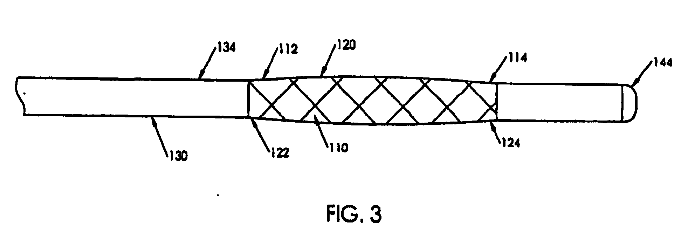

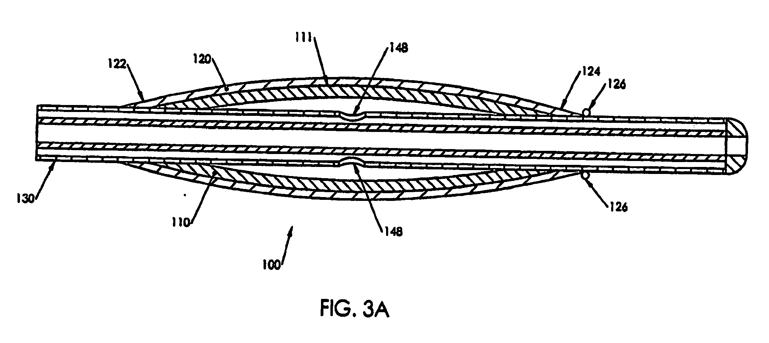

Referring to Figs. 2 and 3, the balloon 110 is located proximate to the distal end 134 of the catheter 130. The balloon 110 has a proximal balloon end 112, a distal balloon end 114, and an interior cavity 116 located between the proximal balloon end 112 and the distal balloon end 114. Preferably, the balloon 110 is disposed circumferentially about the distal end 134 of the catheter 130 and is located proximally of the distal tip 144 of the catheter 130. Preferably, the balloon 110 is constructed of polyurethane, silicone or some other suitable biocompatible material.

The proximal balloon end 112 and the distal balloon end 114 are preferably fixedly attached to the exterior wall 142 of the catheter 130, by bonding, adhesion, ultrasonic welding or any other attachment means that is suitable to fixedly attach the proximal balloon end 112 and the distal balloon end 114 to the exterior wall 142 of the catheter 130. Preferably, the fixed attachment of the proximal and distal balloon ends 112, 114 to the catheter 130 forms a liquid-tight seal between the balloon 110 and the catheter 130. Preferably, in its deflated state, as shown in Fig. 3, the balloon 110 is generally tubular in shape, extending between the proximal balloon end 112 and the distal balloon end 114. As to size, the balloon could have a diameter of from 4 to 20 mm for use in the venous system, and of from 1.5 to 12 mm for use in the arterial system.

Referring back to Figs. 2 and 3, preferably the proximal balloon end 112 and the distal balloon end 114 are shaped to conform to the outside surface of the distal end 134 of the catheter 130. In a case such as the present embodiment, wherein the catheter 130 is a co-axial catheter having a circular outer profile, the proximal and distal balloon ends 112, 114 would have circular profiles and be sized to fit about the exterior wall 142 of the catheter 130. However, those skilled in the art will recognize that the catheter 130 and, correspondingly, the proximal and distal balloon ends 112, 114 may have any shape that facilitates the insertion and operation of the present invention.

Referring now to Fig. 2, an inner surface 111 of the balloon and the exterior wall 142 of the catheter 130 define the interior cavity 116 that is in fluid communication with the exterior lumen 138 of the catheter 130. At least one inflation port 148 is located in the exterior wall 142 of the catheter 130. The at least one inflation port 148 is disposed through the exterior wall 142 of the catheter 130 in the portion of the catheter 130 that is located between the distal balloon end 112 and the proximal balloon end 114. The inflation port 148 facilitates the inflation of the balloon 110 by allowing pressurized fluid to pass from the exterior lumen 138 into the interior cavity 116, thereby expanding the balloon 110.

A covering 120, having an outer surface 121 is disposed about and raised very slightly above the outside of the balloon 110 between a proximal covering end 122 and a distal covering end 124. The covering 120 is preferably constructed of plastic or metal, and may be a mesh of flexible woven polyurethane fibers. However, the covering 120 may be constructed out of any other suitable material, as is known to those skilled in the art to define a covering that is flexible enough to expand when the balloon 110 is inflated and contract when the balloon 110 is deflated.

The covering 120 may have a cross-hatched pattern, wherein the cutting edges are at substantially diagonal angles from the longitudinal and circumferential. Also, those skilled in the art will recognize that the covering 120 may have a longitudinal or a lateral pattern as well. The outer surface 121 of the covering 120 is preferably very sharp, thereby facilitating the laceration of lesions, stenoses or other intended materials within the vessel during use. Preferably, when the balloon 110 and the covering 120 are inflated, the fibers of the covering 120 will create an interlocking structure with an outer surface 121 comprised of sharp individual fibers disposed together in a pattern about the outside of the balloon 110.

The covering 120 may be connected to the balloon 110 at one or more points, or alternatively, the covering 120 may be connected directly to the catheter 130 at one or more points. Alternatively, as shown in Fig. 3a, the covering 120 may be connected to the assembly 100 at one of the proximal and distal covering ends 122, 124. The embodiment shown in Fig. 3a is the same as the preferred embodiment except that in the alternative embodiment shown in Fig. 3a, a longitudinally translatable ring 126 is used to secure at least part of the covering 126 to the assembly 100 and is disposed at the other of the proximal and distal ends 122, 124 of the covering 120. Regardless of how the covering 120 is connected to the assembly 100, it is preferable that there is sufficient flexibility, between the covering 120 and the assembly 100, to allow the balloon 110 to expand when inflated.

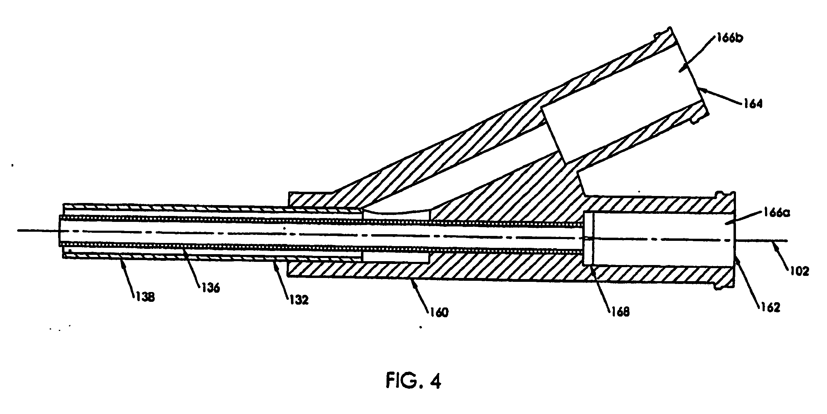

Preferably, as shown in Fig. 4, the proximal end 132 of the catheter 130 comprises a hub 160 that is adapted to mate to the proximal end 132 of the catheter 110 so as to provide fluid communication between the axial lumen 136 and an axial port 162, and between the exterior lumen 138 and an exterior port 164. Preferably, the axial and exterior ports 162, 164 comprise luer fittings 166a, 166b respectively and are capped when the assembly 100 is not in use.

The axial port 162, which is in fluid communication with the axial lumen 136, facilitates the insertion of a guidewire (see Fig. 5) therethrough and comprises a valve 168 disposed therein. The valve 168 restricts the passage of blood, air or contaminants through the axial lumen 162 and is preferably a self sealing valve, and may be an elastomer made from silicone or some other suitable material, as is known to those skilled in the art.

The exterior port 164 is in fluid communication with the exterior lumen 138, and the luer fitting 166b of the exterior port 164 facilitates the connection of the exterior port 164 to an inflation device such as a syringe (not shown) or other suitable mechanical device (also not shown). Such mechanical devices may be an endoflator or other device known to those skilled in the art.

Referring now to Figs. 2 and 5, the cutting balloon assembly 100 may be used to break through the fibrous tissue 149 that comprises lesions within the venous system. The assembly 100 is also intended to be used in expanding a dialysis access, such as a fistula or graft. The cutting balloon assembly 100 may also be used to expand the interior walls of a patient's blood vessels that have been constricted with plaque and stenoses. It is preferable that, prior to insertion into the patient, the exterior lumen 138 of the catheter 130 and the interior cavity 116 of the balloon 110 are primed with a fluid, such as a saline solution Also prior to insertion, it is preferable that exterior surface of the assembly 100 be lubricated with a saline solution or some other suitable lubricant.

With reference to Figure 5, in use a guidewire 150 having a distal tip 152 is inserted into a patient's blood vessel 154 using means known to those skilled in the art, far enough that the distal tip 152 extends past the buildup of stenoses, plaque or lesions 149 within the vessel 154. The cutting balloon catheter assembly 100 is then inserted into the patient's vessel 154 by inserting the proximal end (not shown) of the guidewire 150 into the distal passageway 146 of the distal tip 144 of the assembly 100. The proximal end of the guidewire 150 is fed through the axial lumen 136 as the assembly 100 is slid distally along the guidewire 150 into the patient's vessel 154. As the assembly 100 is slid distally along the guidewire 150, the distal tip 144 of the assembly 100 enters the patient's blood vessel 154 first, followed by the balloon 110; and the assembly 100 continues to be slid along the guidewire 150 until the balloon 110 and the covering 120 are proximate to stenoses, plaque buildups or other lesions 149 inside of the patient's vessel 154.

Once the balloon 110 and mesh covering 120 are proximate to the stenoses, plaque or lesions 149 that are to be cut or expanded, the balloon 110 is inflated, by injecting fluid into the exterior lumen 138 of the catheter 130 through the exterior port 164, shown in Fig. 4. Referring back to Figs. 2 and 5, the balloon 110 is inflated to a size that is large enough to engage and create pressure on the stenoses, plaque or lesions along the interior walls of the patient's blood vessel 154. Once the balloon 110 is sufficiently inflated, the assembly 100 may be rotated or translated longitudinally (axially), and reciprocally so moved, to help facilitate the mesh surface cutting the stenoses, plaque or lesions 149. Rotating the assembly 100 about the longitudinal axis 102 is preferable when an embodiment of the covering 120 has laterally disposed cutting edges. Translating the assembly 100 back and forth in the proximal and distal directions along the longitudinal axis 102 is preferable when an embodiment of the covering 120 having longitudinally disposed cutting edges is used. In use, the cutting edges of covering 120 are intended to cut into stenoses, plaque or lesions 149 within the vessel 154, there by allowing the vessel 154 to expand and facilitate an increased flow of blood therethrough. Rotating or twirling the assembly would not be suitable were the covering a mesh covering with cutting edges at angles substantially diagonal to the longitudinal and circumferential directions.

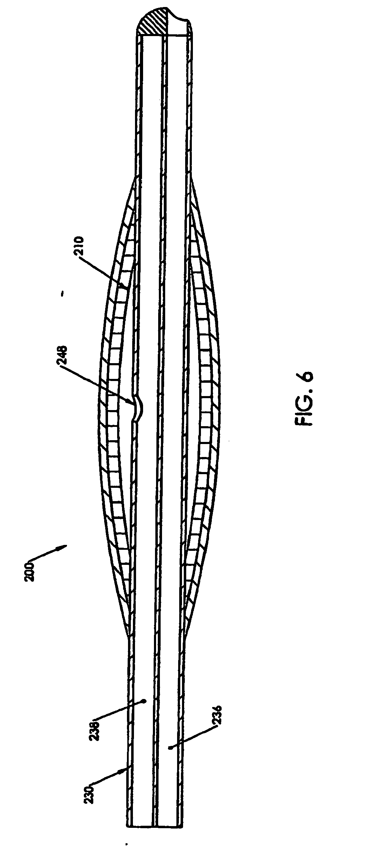

Referring now to Fig. 6, an alternative embodiment of a cutting balloon catheter assembly 200 is shown. The alternative embodiment comprises a catheter 230, with two lumens, having a side-by-side configuration rather than a coaxial design. The catheter 230 with a side-by- , side configuration performs substantially the same function as the coaxial catheter 130 described above. In the side-by-side configuration, a first lumen 236 is adapted to be insertable into a patient's blood vessel, over a guidewire. A second lumen 238 is constructed as a fluid conduit for inflation of a balloon 210. The second lumen 238 is in fluid communication with the balloon 210 through at least one inflation port 248. Those skilled in the art will recognize that this alternative embodiment of the assembly 200 contains, with the exception of the catheter 230 configuration, substantially the same features as the preferred embodiment of the assembly 100. Likewise, this alternative assembly 200 is operated in the same manner as the preferred embodiment 100.

## Claims

1. A cutting balloon catheter assembly (100) for removing material blocking a blood vessel, comprising:
a dual lumen catheter (130) having a distal end (134), a first lumen (138) and a second lumen (136);
an inflatable balloon (110) having an interior cavity (116); and
an expandable covering (120) disposed about the balloon, the covering defining an array of cutting edges (121) that are sufficiency sharp so as to lacerate material of a blockage;
wherein the balloon (110) is fixedly connected to the distal end of the catheter;
wherein the interior cavity of the balloon is in fluid communication with the first lumen (130); and
wherein the second lumen (136) is adapted to be insertable over a guidewire.

2. The cutting balloon catheter assembly (100) according to Claim 1, wherein the dual lumen catheter is a coaxial catheter.

3. The cutting balloon catheter assembly (100) according to Claim 1, wherein the covering (120) comprises a mesh coating of polyurethane fibers.

4. The cutting balloon catheter assembly (100) to Claim 1, wherein the cutting edges (121) of the covering (100) are of a pattern at angles to the longitudinal and circumferential.

5. The cutting balloon catheter assembly (100) according to Claim 1, wherein the cutting edges (121) of the covering (120) are of a pattern that are longitudinal.

6. The cutting balloon catheter assembly (100) according to Claim 1, wherein the cutting edges (121) of the covering (120) are of a pattern that is both longitudinal and circumferential.

7. The cutting balloon catheter assembly (100) according to Claim 1, wherein the cutting edges of the covering (120) extend to outer edges that are very sharp.

8. The cutting balloon catheter assembly (100) according to Claim 1, wherein the covering (120) is of metal.

## Patentansprüche

1. Schneidende Ballonkatheteranordnung (100) zum Entfernen von ein Blutgefäß blockierendem Material, die Folgendes umfasst:
einen Doppellumen-Katheter (130) mit einem distalen Ende (134), einem ersten Lumen (138) und einem zweiten Lumen (136);
einen aufblasbaren Ballon (110) mit einem Innenhohlraum (116); und
eine expandierbare Hülle (120), die um den Ballon angeordnet ist, wobei die Hülle eine Anordnung von Schneidkanten (121) definiert, die ausreichend scharf sind, um Verstopfungsmaterial aufzureißen;
wobei der Ballon (110) fest mit dem distalen Ende des Katheters verbunden ist;
wobei der Innenhohlraum des Ballons in Fluidverbindung mit dem ersten Lumen (138) ist; und
wobei das zweite Lumen (136) so gestaltet ist, dass es über einen Führungsdraht einfügbar ist.

2. Schneidende Ballonkatheterbaugruppe (100) nach Anspruch 1, wobei der Doppellumen-Katheter (130) ein koaxialer Katheter ist.

3. Schneidende Ballonkatheterbaugruppe (100) nach Anspruch 1, wobei die Hülle (120) einen Netzüberzug aus Polyurethanfasern umfasst.

4. Schneidende Ballonkatheterbaugruppe (100) nach Anspruch 1, wobei die Schneidkanten (121) der Hülle (120) ein Muster aus in einem Winkel zur Längs- und Umfangsachse verlaufenden Linien aufweisen.

5. Schneidende Ballonkatheterbaugruppe (100) nach Anspruch 1, wobei die Schneidkanten (121) der Hülle (120) ein in Längsrichtung verlaufendes Muster aufweisen.

6. Schneidende Ballonkatheterbaugruppe (100) nach Anspruch 1, wobei die Schneidkanten (121) der Hülle (120) sowohl ein in Längs- als auch in Umfangsrichtung verlaufendes Muster aufweisen.

7. Schneidende Ballonkatheterbaugruppe (100) nach Anspruch 1, wobei die Schneidkanten (121) der Hülle (120) zu den Außenkanten verlaufen, die sehr scharf sind.

8. Schneidende Ballonkatheterbaugruppe (100) nach Anspruch 1, wobei die Hülle (120) aus Metall ist.

## Revendications

1. Ensemble de cathéter à ballon de découpe (100) pour enlever de la matière bloquant un vaisseau sanguin, comprenant :
un cathéter à double lumière (130) ayant une extrémité distale (134), une première lumière (138) et une seconde lumière (136) ;
un ballon gonflable (110) ayant une cavité intérieure (118) ; et
un revêtement extensible (120) disposé autour du ballon, le revêtement définissant une matrice de bords de découpe (121) qui sont suffisamment tranchants pour lacérer la matière d'un blocage ;
où le ballon (110) est relié de manière fixe à l'extrémité distale du cathéter ;
où la cavité intérieure du ballon est en communication fluide avec la première lumière (138) ; et
où la seconde lumière (136) est adaptée pour être insérable sur un fil guide.

2. Ensemble de cathéter à ballon de découpe (100) selon la Revendication 1, où le cathéter à double lumière (130) est un cathéter coaxial.

3. Ensemble de cathéter à ballon de découpe (100) selon la Revendication 1, où le revêtement (120) comprend un enrobage en maille de fibres de polyuréthane.

4. Ensemble de cathéter à ballon de découpe (100) selon la Revendication 1, où les bords de découpe (121) du revêtement (120) sont d'un modèle à angles par rapport à la longitude et circonférence.

5. Ensemble de cathéter à ballon de découpe (100) selon la Revendication 1, où les bords de découpe (121) du revêtement (120) sont d'un modèle qui est longitudinal.

6. Ensemble de cathéter à ballon de découpe (100) selon la Revendication 1, où les bords de découpe (121) du revêtement (120) sont d'un modèle qui est à la fois longitudinal et circonférentiel.

7. Ensemble de cathéter à ballon de découpe (100) selon la Revendication 1, où les bords de découpe (121) du revêtement (120) s'étirent vers des bords externes qui sont très tranchants.

8. Ensemble de cathéter à ballon de découpe (100) selon la Revendication 1, où le revêtement (120) est en métal.
